# EUROPEAN PATENT SPECIFICATION

(11) **EP 1 621 207 B1**
(45) Date of publication and mention of the grant of the patent: **23.09.2009**
(21) Application number: 05108424.2
(22) Date of filing: 10.04.2001
(51) Int. Cl.: A61K 38/19, A61P 7/00, C12N 5/06, A61K 31/70

(54) **Mixture of defibrotide and G-CSF, and its use for activating haematopoietic progenitors**
Kombination von Defibrotide und G-CSF und ihre Verwendung zur Aktivierung hämatopoietischer Vorläuferzellen
Composition de défibrotide et de G-CSF et son utilisation pour l'activation des progéniteurs hématopoiétiques

(43) Date of publication of application: 01.02.2006
(62) Divisional of application: 01931582.9
(73) Proprietor: Gentium S.p.A., 22079 Villa Guardia (Como) (IT)
(72) Inventor: FERRO, Laura, I-20100, MILANO (MI) (IT); PORTA, Roberto, I-22012, CERNOBBIO (CO) (IT); IACOBELLI, Massimo, I-20152, MILANO (MI) (IT); GIANNI, Alessandro Massimo, I-20135, MILANO (MI) (IT); STELLA, Carmelo Carlo, I-20129, MIlano (MI) (IT)
(74) Representative: Pistolesi, Roberto

(56) References cited:
- EP-A- 0 317 766
- EP-A- 0 416 678
- WO-A-99/26639
- US-A- 5 199 942
- US-A- 5 977 083
- GURSOY A: "PREPARATION, CHARACTERIZATION AND ANTI-INFLAMMATORY EFFECT OF DEFIBROTIDE LIPOSOMES" PHARMAZIE,DD,VEB VERLAG VOLK UND GESUNDHEIT. BERLIN, vol. 48, no. 7, 1 July 1993 (1993-07-01), pages 549-550, XP000372658 ISSN: 0031-7144
- CARLO-STELLA, C. (1) ET AL: "Defibrotide significantly enhances peripheral blood progenitor cell mobilization induced by recombinant human granulocyte colony - stimulating factor ( rhG - CSF." BLOOD, PART 1, PP. 553A. PRINT. MEETING INFO.: 42ND ANNUAL MEETING OF THE AMERICAN SOCIETY OF HEMATOLOGY SAN FRANCISCO, CALIFORNIA, USA DECEMBER 01-05, 2000 AMERICAN SOCIETY OF HEMATOLOGY., vol. 96, no. 11, 16 November 2000 (2000-11-16), XP002176349

## Description

The present invention relates to a novel formulation capable of increasing the amount of stem cells and progenitor cells in circulation in the peripheral blood of a mammal; the formulation is characterised in that it contains defibrotide in combination with at least one haematopoietic factor having the capacity to mobilise haematopoietic progenitors, preferably G-CSF.

### FIELD OF THE INVENTION

The possibility of obtaining an increased amount of stem cells and haematopoietic progenitors in circulation in the peripheral blood of a mammal, and in particular in that of a human being, has for years been the subject of intensive research activity; the availability of stem cells and/or haematopoietic progenitors is in fact particularly important in sectors such as the autologous transplantation of circulating haematopoietic progenitors, the allotransplantation of circulating haematopoietic progenitors and in program for the gene therapy of circulating haematopoietic cells.

Although, after birth, stem cells and progenitor cells are located almost exclusively in the bone marrow, they nevertheless exhibit migratory properties; that is to say, under physiological conditions, they migrate through the cavities of the bone marrow and pass into circulation. That process, commonly known as "mobilisation", can be amplified in mammals by various treatments, such as, for example, the administration of cytokines and, in particular, the growth factor of granulocyte colonies (G-CSF); the reverse process, known as "homing", occurs, for example, in irradiated receivers after the transplantation of haematopoietic cells; the mechanisms on which mobilisation and homing are based are, however, still obscure (C.F. Craddock et al., "Antibodies to VLA4 Integrin Mobilize Long-Term Repopulating Cells and Augment Cytokine-Induced Mobilization in Primates and Mice", Blood, Vol. 90, n. 12, 1997, pp. 4779-4788; F. Prosper et al., "Mobilization and Homing of Peripheral Blood Progenitors is Related to Reversible Downregulation of α4β1 Integrin Expression and Function", J. Clin. Invest., Vol. 101, n. 11, 1998, pp. 2456-2467; M. Vermeulen et al., "Role of Adhesion Molecules in the Homing and Mobilization of Murine Hematopoietic Stem and Progenitor Cells", Blood, Vol. 92, n.3, 1998, pp. 894-900).

G-CSF (CAS registry number 143011-2-7/Merck Index, 1996, page 4558) is a haematopoietic growth factor which is indispensable in the proliferation and differentiation of the progenitor cells of granulocytes; it is a 18-22 kDa glycoprotein normally produced in response to specific stimulation by a variety of cells, including monocytes, fibroblasts and endothelial cells. The term defibrotide (CAS registry number 83712-60-1) normally identifies a polydeoxyribonucleotide obtained by extraction (US 3,770,720 and US 3,899,481) from animal and/or vegetable tissue; this polydeoxyribonucleotide is normally used in the form of a salt of an alkali metal, generally sodium. Defibrotide is used principally for its antithrombotic activity (US 3,829,567) although it may be used in different applications, such as, for example, the treatment of acute renal insufficiency (US 4,694,134) and the treatment of acute myocardial ischaemia (US 4,693,995). United States patents US 4,985,552 and US 5,223,609, finally, describe a process for the production of defibrotide which enables a product to be obtained which has constant and well defined physico-chemical characteristics and is also free from any undesired side-effects.

For the purposes of the present invention, the term defibrotide should therefore be understood as meaning any oligonucleotide and/or polynucleotide obtained by extraction from animal and/or vegetable tissue, in particular from the organs of mammals. Preferably, defibrotide is produced in accordance with the methods described in the patents listed above which should thus be regarded as an integral part of the present description; even more preferably, it is produced in accordance with the method described in United States patents US 4,985,552 and US 5,223,609.

### DETAILED DESCRIPTION OF THE INVENTION

It has now surprisingly been found that it is possible to obtain increased mobilisation of stem cells and haematopoietic progenitors by the administration of defibrotide in combination and/or in close temporal proximity with a haematopoietic factor having the capacity to mobilise haematopoietic progenitors.

As will be appreciated from the Examples, the administration of defibrotide in combination and/or in close temporal proximity with a haematopoietic factor having the capacity to mobilise haematopoietic progenitors permits the attainment of mobilisation levels much higher than those obtainable by the administration of the haematopoietic factor alone.

The subject of the present invention is therefore represented by a formulation containing as "active agents" defibrotide in combination with at least one haematopoietic factor having the capacity to mobilise haematopoietic progenitors, preferably G-CSF. In its preferred embodiment, this formulation is constituted by an injectable aqueous solution; alternatively, the formulation could be constituted by two different solutions, one containing defibrotide and the other containing the haematopoietic factor having the capacity to mobilise haematopoietic progenitors.

The formulation according to the present invention is therefore shaped as a combined preparation for simultaneous, separate or sequential use of the aforementioned active principles in order to increase the amount of stem cells and/or haematopoietic progenitors in circulation in the peripherial blood of a mammal.

A second subject of the present invention is represented by the use of defibrotide, in combination with at least one haematopoietic factor having the capacity to mobilise haematopoietic progenitors, for the preparation of formulations capable of increasing the amount of stem cells and/or haematopoietic progenitors in circulation in the peripheral blood of a mammal, preferably a human being.

Finally, a further subject of the present invention is represented by a method of increasing the amount of stem cells and/or haematopoietic progenitors in circulation in the peripheral blood of a mammal, characterised in that defibrotide is administered to the mammal in combination or in temporal proximity with at least one haematopoietic factor having the capacity to mobilise haematopoietic progenitors.

The haematopoietic factor used to conduct the experiments which led to the present invention is G-CSF; however, it is not to be excluded that similar results may be obtained with haematopoietic factors other than G-CSF but nevertheless having the capacity to mobilise haematopoietic progenitors, such as, for example, the growth factor of granulocyte and macrophage colonies (GM-CSF), "Flt3 ligand" (FL), "stem cell factor" (SCF), thrombopoietin (TPO), interleukin 8 (IL-8), and others which will be clear to persons skilled in the art.

The defibrotide used in combination with G-CSF in this first experimental stage was the defibrotide currently marketed by Crinos Spa under the mark Prociclide™ and produced in accordance with the process described in United States patents US 4,985,552 and US 5,223,609.

As regards the methods of administering the two active ingredients, they are not limiting for the purposes of the invention. That is to say, defibrotide and haematopoietic factor having the capacity to mobilise haematopoietic progenitors can be administered to mammals (and in particular to human beings) in accordance with the methods and the posologies known in the art; generally, they are administered orally, intramuscularly, intraperitoneally, subcutaneously or intravenously, the last-mentioned route being the preferred one.

The two active ingredients can also be administered simultaneously or in succession. That is to say, in the first case, they are administered by means of a single formulation which contains both of the active ingredients and to which the usual excipients and/or coadjuvants known in the art have optionally been added; alternatively, the two active ingredients may be administered sequentially, namely, by means of two different formulations, one containing the haematopoietic factor having the capacity to mobilise haematopoietic progenitors, preferably G-CSF, and the other containing the defibrotide.

Generally, G-CSF will be administered subcutaneously, at a dosage of 5 to 24 µg/kg whereas DEF will be administered by continuous infusion at a dosage of 5 to 15 mg/kg/hr for 2-7 days.

As will be appreciated from the accompanying Examples, which are to be regarded purely as non-limiting illustrations of the invention, the combined administration of G-CSF and defibrotide to mice, as the most common experimental mammal model, and to monkeys, permits the attainment of levels of mobilisation much higher than those obtainable by the administration of G-CSF alone, with clear advantages for all those therapeutic sectors for which a high level of mobilisation is desirable.

### EXAMPLE 1

This experiment was carried out to evaluate the effect of the administration of G-CSF and/or defibrotide (DEF) on the amount of white blood cells (WBC) present in murine blood. BALB/c mice from 6 to 8 weeks old and having a body weight of from 20 to 25 g were subjected to intraperitoneal (IP) injections of G-CSF (5 µg/mouse/day), DEF (1 mg/mouse/day), or a combination of G-CSF (5 µg /mouse/day) and increasing doses of DEF (1, 10, 15 mg/mouse/day). A saline solution, buffered to 0.1%, of murine serum albumin (PBS/MSA) was administered by IP injection to the control mice which had not received G-CSF and/or DEF. The mice were treated for 5 days and sacrificed after 3 or 5 days of treatment, or 3 days after therapy had ceased.

### EXAMPLE 2

Mobilisation kinetics of the cells forming the total colonies (CFC) per millilitre of blood of the mice of Example 1. The mean CFC count in PBS/MSA in the control mice was 39 ± 12 per ml of blood.

### EXAMPLE 3

Changes in the frequency of the total CFCs (CFU-GM + BFU-E + CFU-Mix + HPP-CFC) per 10⁵ buffy-coat cells of peripheral blood in the mice mentioned in Example 1. The mean CFC count in PBS/MSA in the control mice was 3.5 ± 1.

### EXAMPLE 4

Total CFCs (CFU-GM + BFU-E + CFU-Mix + HPP-CFC) per millilitre of blood after 5 days' treatment of the mice of Example 1.

### EXAMPLE 5

Frequency of the total CFCs (CFU-GM + BFU-E + CFU-Mix + HPP-CFC) for 10⁵ buffy -coat cells of peripheral blood in the mice mentioned in Example 1.

### EXAMPLE 6

This experiment was carried out to evaluate the effect of the administration of G-CSF and/or DEF on the amount of haematopoietic progenitors/stem cells (High-Proliferative Potentia-Colony Stimulating Cells, or HPP-CFC) in circulation in the peripheral blood of monkeys. The study was carried out in rhesus monkeys (Macaca Mulatta) of 4-6 years in good health and which showed normal values for hematology and clinical chemistry. The G-CSF was dosed subcutaneously, 100 µg/kg, for 5 days for two cycles; DEF was dosed 15 mg/kg/hr by a continuous infusion system for 5 days at the second cycle. The animals were anaesthetised for bleeding, administration of G-CSF and changing the drug-bag. The results of this experiment show that the combined administration of G-CSF and defibrotide enables an HPP-CFC mobilisation in monkeys which is about 8 times higher than that obtainable by the administration of G-CSF alone.

### CONCLUSIONS

The combined administration of G-CSF and defibrotide produces a substantial increase in the amount of white blood cells in circulation in murine blood. It should, in particular, be noted that the administration of defibrotide alone does not have a positive influence on the amount of white blood cells in circulation; the combination of G-CSF and defibrotide therefore produces a surprising dose-dependent effect of increasing the number of white blood cells, which is not merely the sum of two effects which are independent of one another.

The combined administration of G-CSF and defibrotide enables levels of mobilisation to be obtained in mice that are from 10 to 100 times higher than those obtainable by the administration of G-CSF alone.

The combined administration of G-CSF and defibrotide enables a stem cell (HPP-CFC) mobilisation in monkeys which is about 8 times higher than that obtainable by the administration of G-CSF alone.

The combined effect of the two active ingredients is dose-dependent because the levels of mobilisation increase proportionally with the amount of defibrotide administered; the highest mobilisation peak is reached in all cases on approximately the fifth day from administration.

## Claims

1. Defibrotide in combination with a haematopoietic factor having the capacity to mobilise haematopoietic progenitors, for use in increasing the amount of stem cells and/or haematopoietic progenitors in circulation in the peripheral blood of a mammal, **characterised in that** defibrotide and said at least one haematopoietic factor having the capacity to mobilise haematopoietic progenitors are administered intravenously.

2. Defibrotide for use according to claim 1, **characterised in that** the haematopoietic factor having the capacity to mobilise haematopoietic progenitors is selected from G-CSF, GM-CSF, FL, SCF, TPO and/or IL-8.

3. Defibrotide for use according to claim 2, **characterised in that** the haematapoietic factor having the capacity to mobilise haematopoietic progenitors is G-CSF.

4. Defibrotide for use according to claim 3. **characterised in that** G-CSF is administered at a dosage of 5 to 24 µg/kg and defibrotide is administered at a dosage of 5 to 15 mg/kg/hr.

5. Defibrotide for use according to claim 4, **characterised in that** G-CSF and defibrotide are administered for 2-7 days.

6. Defibrotide for use according to any of the above claims, **characterised in that** the mammal is a human being.

7. Defibrotide for use according to any of the above claims, **characterised in that** defibrotide and said at least one haematopoietic factor having the capacity to mobilise haematopoietic progenitors are administered simultaneously or in succession.

## Patentansprüche

1. Défibrotide in Kombination mit einem haematopoietischen Faktor mit der Eigenschaft haematopoietische Vorläuferzellen zu mobilisieren, zur Verwendung bei der Erhöhung der Anzahl von Stammzellen und/oder haematopoietichen Vorläuferzellen im peripheren Blutkreislauf eines Säugetiers, **dadurch gekennzeichnet, dass** Defibrotide und der wenigstens eine haematopoietische Faktor mit der Eigenschaft haematopoietische Vorläuferzellen zu mobilisieren intravenös verabreicht werden.

2. Defibrotide zur Verwendung nach Anspruch 1, **dadurch gekennzeichnet, dass** der haematopoietische Faktor mit der Eigenschaft haematopoietische Vorläuferzellen zu mobilisieren aus G-CSF, GM-CSF, FL, SCF, TPO und/oder IL-8 gewählt ist.

3. Defibrotide zur Verwendung nach Anspruch 2, **dadurch gekennzeichnet, dass** der haematopoietische Faktor mit der Eigenschaft haematopoietische Vorläuferzellen zu mobilisieren G-CSF ist.

4. Defibrotide zur Verwendung nach Aspruch 3, **dadurch gekennzeichnet, dass** G-CSF in einer Dosierung von 5 bis 24 µg/kg und Defibrotide in einer Dosierung von 5 bis 15 mg/kg/hr verabreicht wird.

5. Defibrotide zur Verwendung nach Anspruch 4, **dadurch gekennzeichnet, dass** G-CSF und Defibrotide für 2 bis 7 Tage verabreicht wird.

6. Defibrotide zur Verwendung nach einem der vorangegangen Ansprüche, **dadurch gekennzeichnet, dass** das Säugetier ein Mensch ist.

7. Défibrotide zur Verwendung nach einem der vorangegangen Ansprüche, **dadurch gekennzeichnet, dass** Defibrotide und der wenigstens eine haematopoeietische Faktor mit der Eigenschaft haematopoietische Vorläuferzellen zu mobilisieren gleichzeitig oder nacheinander verabreicht werden.

## Revendications

1. Défibrotide en combinaison avec un facteur hématopoïétique ayant la capacité de mobiliser des progéniteurs hématopoïétiques utilisable pour augmenter la quantité de cellules souches et/ou des progéniteurs hématopoïétiques circulant dans le sang périphérique d'un mammifère **caractérisé en ce que** le défibrotide et ledit au moins un facteur hématopoïétique ayant la capacité de mobiliser des progéniteurs hématopoïétiques sont administrés par intraveineuse.

2. Défibrotide devant être utilisé selon la revendication 1, **caractérisé en ce que** le facteur hématopoïétique ayant la capacité de mobiliser des progéniteurs hématopoïétiques est choisi parmi G-CSF, GM-CSF, FL, CSF, TPO et/ou IL-8.

3. Defibrotide devant être utilisé selon la revendication 2, **caractérisé en ce que** le facteur hématopoïétique ayant la capacité de mobiliser des progéniteurs hématopoïétiques est G-CSF.

4. Défibrotide devant être utilisé selon la revendication 3, **caractérisé en ce que** le G-CSF est administré avec un dosage de 5 à 24 µg/kg et le défibrotide est administré avec un dosage de 5 à 15 mg/kg/h.

5. Défibrotide devant être utilisé selon la revendication 4, **caractérisé en ce que** le G-CSF et le défibrotide sont administrés pendant 2 à 7 jours.

6. Défibrotide devant être utilisé selon l'une des revendications précédentes, **caractérisé en ce que** le mammifère est un être humain.

7. Défibrotide devant être utilisé selon l'une des revendications précédentes, **caractérisé en ce que** le défibrotide et ledit au moins un facteur hématopoïétique ayant la capacité de mobiliser des progéniteurs hématopoïétiques sont administrés simultanément ou successivement.
